# EUROPÄISCHE PATENTANMELDUNG

(11) **EP 1 403 241 A1**
(43) Veröffentlichungstag der Anmeldung: **31.03.2004**
(21) Anmeldenummer: 02021630.5
(22) Anmeldetag: 27.09.2002
(51) Int. Cl.: C07C 209/78, C07C 211/49

(54) **Verfahren zur Herstellung von 4,4'-Diaminoditolylmethan**

(71) Anmelder: BASF AKTIENGESELLSCHAFT, 67056 Ludwigshafen (DE)
(72) Erfinder: Ströfer, Eckhard, Dr., 68163 Mannheim (DE); Sohn, Martin, Dr., 68229 Mannheim (DE); Langensiepen, Hans-Werner, Dr., 3080 Tervuren (BE); Müller, Christian, 68167 Mannheim (DE); Krug, Georg, 69509 Mörlenbach (DE); Kemper, Reinhard, Dr., 69123 Heidelberg (DE)

(57) **Zusammenfassung**

Gegenstand der Erfindung ist ein Verfahren zur Herstellung von 4,4'-Diaminoditolylmethan durch Umsetzung von o-Toluidin mit Formaldehyd in Gegenwart eines sauren Katalysators, wobei das Molverhältnis des sauren Katalysators zu o-Toluidin von 0,8 bis 1,2 Äquivalenten des sauren Katalysators pro Mol o-Toluidin beträgt.

Das Molverhältnis o-Toluidin zu Formaldehyd beträgt vorzugsweise von 1,5:1 bis 10:1. Der saure Katalysator ist bevorzugt Salzsäure.

## Beschreibung

Die Erfindung betrifft ein Verfahren zur Herstellung von 4,4`-Diaminoditolylmethan.

Es ist bekannt, 4,4'-Diaminoditolylmethan durch Umsetzung von o-Toluidin mit Formaldehyd in Gegenwart einer Säure als Katalysator herzustellen.

DE-A 1 643 249 beschreibt ein Verfahren zur kontinuierlichen Herstellung von Diaminoditolylmethanen durch Umsetzung von o-Toluidin mit Formaldehyd in Gegenwart von wässriger Mineralsäure und eines mit Wasser nicht mischbaren Lösungsmittels bei erhöhter Temperatur in mehreren hintereinander geschalteten Reaktionsstufen, wobei jede folgende Reaktionsstufe bei einer höheren Temperatur als die vorangegangene Reaktionsstufe durchgeführt wird, Neutralisieren, Abtrennung der wässrigen Phase und Isolierung der gebildeten Diaminotolylmethane. o-Toluidin und Formaldehyd werden im Molverhältnis von 1,8:1 bis 8,0:1 eingesetzt. Die Säure wird in Mengen von 0,5 bis 35 mol-%, bezogen auf ein mol o-Toluidin, eingesetzt. Dem Reaktionsgemisch werden vor oder nach der Umsetzung, also vor der Neutralisierung des Reaktionsgemisches mit Wasser, nicht mischbare organische Lösungsmittel in Mengen von 10 bis 200 Gew.-%, bezogen auf o-Toluidin, zugesetzt.

Nachteilig an dem beschriebenen Verfahren ist, daß neben dem gewünschten Wertprodukt 4,4'-Diaminoditolylmethan signifikante Mengen 2,4'-Diaminoditolylmethan als Nebenprodukt gebildet werden. Die Trennung von 4,4'- und 2,4'-Diaminoditolylmethan durch Destillation oder Kristallisation ist sehr aufwendig, weshalb eine Minimierung der Bildung des 2,4'-Isomers wünschenswert ist.

Aufgabe der Erfindung ist es, ein Verfahren zur Herstellung von 4,4'-Diaminoditolylmethan bereitzustellen, bei dem Nebenprodukte in verringertem Umfang gebildet werden.

Gelöst wird die Aufgabe durch ein Verfahren zur Herstellung von 4,4'-Diaminoditolylmethan durch Umsetzung von o-Toluidin mit Formaldehyd in Gegenwart eines sauren Katalysators, das dadurch gekennzeichnet ist, daß das Molverhältnis des sauren Katalysators zu o-Toluidin von 0,8 bis 1,2 Äquivalenten des sauren Katalysators pro mol o-Toluidin beträgt.

Als saure Katalysatoren können Säuren mit einem pKₛ-Wert < 1,5 eingesetzt werden, beispielsweise Mineralsäuren wie Phosphorsäure, Schwefelsäure und/oder Salzsäure, bevorzugt wird Salzsäure eingesetzt. Bevorzugt beträgt das Molverhältnis des sauren Katalysators zu o-Toluidin von 0,9 bis 1,1 Äquivalenten, besonders bevorzugt von 0,9 bis 1,0 Äquivalenten des sauren Katalysators pro mol o-Toluidin. Wegen der möglichen Bildung von stark karzinogenem Bis-Chlormethylether ist bei Verwendung von Salzsäure als Säure das Arbeiten mit überstöchiometrischen Säuremengen weniger bevorzugt.

Das Molverhältnis von o-Toluidin zu Formaldehyd beträgt üblicherweise von 1,5:1 bis 10:1, bevorzugt von 2,5:1 bis 8,0:1, besonders bevorzugt von 2,5 : 1 bis 5,0 : 1. Durch den Einsatz von o-Toluidin in stöchiometrischem Überschuß wird die Bildung von oligomeren.

o-Toluidin-Formaldehyd-Kondensaten zurückgedrängt. Insbesondere wird die Bildung von N-Methyl-4,4'-Diaminoditolylmethan zurückgedrängt.

Das erfindungsgemäße Verfahren wird bevorzugt halbkontinuierlich durchgeführt, d.h. o-Toluidin und gegebenenfalls eine Teilmenge oder die Gesamtmenge des sauren Katalysators werden vorgelegt und Formaldeyhd und gegebenenfalls die Gesamtmenge oder die Restmenge des sauren Katalysators kontinuierlich zugegeben. Vorzugsweise wird o-Toluidin und die Gesamtmenge des sauren Katalysators vorgelegt. Die Umsetzung wird üblicherweise bei Temperaturen von 20 bis 200°C durchgeführt.

Die Umsetzung kann in einem inerten organischen Lösungsmittel durchgeführt werden. Vorzugsweise wird sie in Abwesenheit eines organischen Lösungsmittels durchgeführt.

In einer besonders bevorzugten Ausführungsform des erfindungsgemäßen Verfahrens wird die Reaktionsmischung während der Formaldehyd-Zugabe, bei einer Temperatur von 20 bis 70°C, bevorzugt von 20 bis 60°C und besonders bevorzugt von 30 bis 60°C gehalten, bis mindestens 50 % der Formaldehydmenge zugegeben sind. Bevorzugt wird die Reaktionsmischung auf der angegebenen Temperatur gehalten, bis die Gesamtmenge Formaldehyd zugegeben ist. Die Temperatur der Reaktionsmischung während der Formaldehyd-Zugabe hat einen Einfluß auf die Isomerenverteilung des gebildeten Diaminoditolylmethans. Anschließend wird die Temperatur auf > 75°C erhöht.

Die Reaktionsmischung wird nach Zugabe von mindestens 50 % der Formaldehydmenge, bevorzugt nach vollständiger Zugabe des Formaldehyds, bevorzugt für eine Dauer von mindestens 0,2 Stunden, besonders bevorzugt 0,2 bis 48 Stunden, insbesondere 0,2 bis 6 Stunden auf einer Temperatur von > 75°C, bevorzugt > 90°C, besonders bevorzugt 100 bis 130 °C, gehalten.

Bei der Temperierung auf Temperaturen > 75°C werden in der Reaktionsmischung unerwünschte Nebenprodukte in einem Alterungsprozeß abgebaut oder umgelagert. Die Alterung der Reaktionsmischung kann in dem Reaktor, in der die Umsetzung von o-Toluidin mit Formaldehyd durchgeführt wurde, ablaufen, oder diskontinuierlich oder kontinuierlich in einem weiteren Reaktor, in das die Reaktionsmischung nach vollständiger Zugabe des Formaldehyds überführt wird. Bevorzugt wird die Reaktionsmischung nach vollständiger Zugabe des Formaldehyd in einen anderen Reaktor überführt.

Die Reaktionsmischung enthaltend 4,4'-Diaminoditolylmethan, oligomere Kondensationsprodukte sowie unumgesetztes o-Toluidin kann in üblicher Weise durch Neutralisation, Phasentrennung und Destillation und/oder chromatographische Trennung der Reaktionsprodukte aufgearbeitet werden. Bevorzugt wird das Reaktionsgemisch mit wässriger Natronlauge neutralisiert und anschließend die wässrige Phase abgetrennt. Zur Entfernung anorganischer Verunreinigungen wird vorzugsweise mehrmals mit Wasser gewaschen. Anschließend wird das Produktgemisch destillativ aufgetrennt.

In einer speziell bevorzugten Ausführungsform des erfindungsgemäßen Verfahrens werden in einer Apparatur o-Toluidin und Mineralsäure vorgelegt, gemischt, in einem Kreislauf bewegt, beispielsweise durch eine übliche Pumpe, und diesem Gemisch, bevorzugt über eine Reaktionsmischpumpe oder Düse, Formaldehyd zugegeben. Die Zugabe des Formaldehyds kann derart erfolgen, daß ein konstanter Volumenstrom in das Reaktionsgemisch eingespeist wird, bis ein geeignetes Molverhältnis von o-Toluidin zu Formaldehyd vorliegt. Bevorzugt erfolgt die Zugabe derart, daß pro Minute 0,05 bis 2 % des o-Toluidin-Volumens in die Reaktionsmischung eingeleitet werden. Der Formaldehyd kann auch derart eingeleitet werden, daß das pro Zeiteinheit zugegebene Volumen gemäß einer mathematischen Funktion mit fortschreitender Zugabe sinkt. Bevorzugt ist dabei eine konstante, linear fallende oder stufig fallende Zugaberate.
Der Formaldehyd kann auch gepulst in die Reaktionsmischung eingeleitet werden, wobei eine regelmäßige oder unregelmäßige Pulsfrequenz und Zugabemenge gewählt werden kann.

Die kreisläufige Bewegung der Reaktionsmischung in der Apparatur kann durch allgemein übliche Einreichungen wie Pumpen erfolgen. Die Geschwindigkeit, mit der die Reaktionsmischung in dem Kreislauf bewegt wird, beträgt bevorzugt 1 bis 6 m/sec. Die Einspeisung der Formaldehydlösung kann über eine Reaktionsmischpumpe, wie in DE-A 42 20 239 beschrieben, oder über ein in den Pumpenkreislauf eingebautes Düsensystem, z.B. einer Ringspaltdüse, erfolgen. Die in der Mischzone des Mischorgans, also der Düse oder der Reaktionsmischpumpe, bei der Einspeisung des Formaldehyds lokal dissipierte Mischenergie beträgt vorzugsweise 100 bis 100 000 W/1. Der in dem Kreislauf umgepumpte Mengenstrom steht zu dem in den Kreislauf eingespeisten Mengenstrom an Formaldehydlösung in einem Verhältnis von bevorzugt mindestens 20:1. Die Reaktionswärme der exothermen Reaktion wird in diesem oder vorzugsweise in einem separaten Umpumpkreislauf abgeführt.

Das Verfahrensprodukt enthält neben 4,4'-Diaminoditolylmethan in geringen Mengen 2,4'-Diaminoditolylmethan, in sehr geringen Mengen 2,2'-Diaminoditolylmethan, daneben oligomere Kondensationsprodukte sowie gegebenenfalls unumgesetzte o-Toluidin.
Der Anteil an gebildetem 2,4'-Diaminoditolylmethan beträgt, bei einem Molverhältnis des sauren Katalysators zu o-Toluidin von 0,98, bezogen auf das 4,4'-Isomere, im allgemeinen < 1,0 Gew.-%, bevorzugt < 0,1 Gew.-%.

Die Erfindung wird durch das nachstehende Beispiel näher erläutert.

### Beispiele

### Beispiel 1 bis 7

In einem 2 l-Rührbehälter mit Umpumpkreislauf und Rückflußkühler werden 400 g (3,72 mol) o-Toluidin (Reinheit > 99,5 %) vorgelegt und bei 60°C mit 435,6 g 30 gew.-%-iger wässriger Salzsäure (3,58 mol) versetzt, entsprechend einem Säure/o-Toluidin-Verhältnis von 0,98. Bei dieser Temperatur wird über einen Zeitraum von 60 Minuten die in der Tabelle angegebene Menge einer 37 gew.-%-igen wässrigen Formaldehydlösung zudosiert. Nach Beendigung der Formaldehyd-Zugabe wird die Temperatur innerhalb von 30 Minuten linear auf 95°C erhöht und 240 Minuten bei 95°C gehalten. Anschließend wird zur Neutralisation ein Überschuß 25 gew.-%-ige NaOH der Reaktionsmischung zugegeben, wobei das Reaktionsprodukt als Feststoff ausfällt. Das Produkt wird zur Entfernung von NaCl mehrmals mit Wasser gewaschen. Der salzfrei gewaschene Roh-Austrag wird anschließend destillativ aufgearbeitet. Dabei wird zunächst Wasser und anschließend o-Toluidin abdestilliert. Die Reindestillation von 4,4`-Diaminoditolylmethan wird bei 260°C Sumpftemperatur und 0,2 bis 0,3 mbar durchgeführt. Es wird eine Leichtsieder-Fraktion, im wesentlichen bestehend aus o-Toluidin, eine Mittelsieder-Fraktion, im wesentlichen bestehend aus 4,4'- und 2,4`-Diaminoditolylmethan, und ein oligomerer Destillationsrückstand erhalten.

### Vergleichsbeispiel V1

Beispiel 6 wurde wiederholt, wobei das Molverhältnis Säure/o-Toluidin nur 0,4 betrug.

Die Zusammensetzung der Produktmischung in Abhängigkeit des gewählten o-Toluidin-Formaldehyd-Verhältnisses wird durch die nachstehende Tabelle wiedergegeben.

| **Beispiel Nr.** | **HCHO- Menge [mol]** | **o-Toluidin/ HCHO- Verhältnis** | **4,4'-Isomer [Gew.-%]** | **2,4'-Isomer [Gew.-%]** | **Sonstige* [Gew.-%]** | **Rückstand [Gew.-%]** |
|---|---|---|---|---|---|---|
| 1 | 1,83 | 2,0 | 73,4 | 0,60 | 0,50 | 21,6 |
| 2 | 1,46 | 2,5 | 75,4 | 0,5 | 0,56 | 21,4 |
| 3 | 1,33 | 2,75 | 83,0 | 0,44 | 0,17 | 14,7 |
| 4 | 1,22 | 3,0 | 84,4 | 0,40 | 0,23 | 8,44 |
| 5 | 1,13 | 3,25 | 88,1 | 0,29 | 0,37 | 9,71 |
| 6 | 1,05 | 3,5 | 90,5 | - | 0,18 | 8,08 |
| 7 | 0,914 | 4,0 | 89,8 | - | 0,25 | 8,16 |
| V1 | 1,05 | 3,5 | 82,6 | 3,23 | 0,05 | 12,0 |

| | | | | | | |
|---|---|---|---|---|---|---|
| * unbekannte Peaks im Gaschromatogramm = leichtsiedende Nebenprodukte | | | | | | |

Gemäß Vergleichsbeispiel V1 wird deutlich mehr des 2,4'-Isomeren und deutlich weniger des 4,4'-Isomeren gebildet.

## Patentansprüche

1. Verfahren zur Herstellung von 4,4'-Diaminoditolylmethan durch Umsetzung von o-Toluidin mit Formaldehyd in Gegenwart eines sauren Katalysators, **dadurch gekennzeichnet, daß** das Molverhältnis des sauren Katalysators zu o-Toluidin von 0,8 bis 1,2 Äquivalenten des sauren Katalysators pro Mol o-Toluidin beträgt.

2. Verfahren nach Anspruch 1, **dadurch gekennzeichnet, daß** das Molverhältnis o-Toluidin zu Formaldehyd von 1,5:1 bis 10:1 beträgt.

3. Verfahren nach Anspruch 1 oder 2, **dadurch gekennzeichnet, daß** der saure Katalysator Salzsäure ist.

4. Verfahren nach einem der Ansprüche 1 bis 3, **dadurch gekennzeichnet, daß** das Verfahren halbkontinuierlich durchgeführt wird, indem o-Toluidin und gegebenenfalls eine Teilmenge oder die Gesamtmenge des sauren Katalysator vorgelegt werden und Formaldehyd und gegebenenfalls die Restmenge des sauren Katalysators kontinuierlich zugegeben werden.

5. Verfahren nach einem der Ansprüche 1 oder 4, **dadurch gekennzeichnet, daß** die Temperatur der Reaktionsmischung während der Formaldehyd-Zugabe bei einer Temperatur von 20 bis 70°C gehalten und anschließend auf eine Temperatur oberhalb 75°C erhöht wird.

## Geänderte Patentansprüche

### Geänderte Patentansprüche gemäss Regel 86(2) EPÜ.

**1.** Verfahren zur Herstellung von 4,4'-Diaminotolylmethan durch Umsetzung von o-Toluidin mit Formaldehyd in Gegenwart eines sauren Katalysators, **dadurch gekennzeichnet, daß** man halbkontinuierlich
o-Toluidin und gegebenenfalls eine Teilmenge oder die Gesamtmenge des sauren Katalysators vorlegt und dieses Gemisch in einem Kreislauf bewegt und
zu diesem Gemisch Formaldehyd und gegebenenfalls die Restmenge des sauren Katalysators gibt,
wobei man die Reaktionsmischung während der Formaldehydzugabe bei einer Temperatur von 20 bis 70°C hält, bis mindestens 50 % der Formaldehydmenge zugegeben sind, und
anschließend das Reaktionsgemisch bei einer erhöhten Temperatur von mehr als 75 °C nachbehandelt.

**2.** Verfahren nach Anspruch 1, **dadurch gekennzeichnet, daß** man die Reaktionsmischung während der Zugabe der Gesamtmenge Formaldehyd bei einer Temperatur von 20 bis 70°C hält und anschließend das Reaktionsgemisch bei einer erhöhten Temperatur von mehr als 75°C nachbehandelt.

**3.** Verfahren nach Anspruch 1 oder 2, **dadurch gekennzeichnet, daß** man die Gesamtmenge des sauren Katalysators vorlegt.

**4.** Verfahren nach einem der vorstehenden Ansprüche, **dadurch gekennzeichnet, daß** das Molverhältnis des sauren Katalysators zu o-Toluidin von 0,9 bis 1,1 Äquivalenten beträgt.

**5.** Verfahren nach einem der vorstehenden Ansprüche, **dadurch gekennzeichnet, daß** das Molverhältnis von o-Toluidin zu Formaldehyd 1,5:1 bis 10:1 beträgt.

**6.** Verfahren nach einem der vorstehenden Ansprüche, **dadurch gekennzeichnet, daß** man bei einer erhöhten Temperatur von mehr als 90 °C nachbehandelt.

**7.** Verfahren nach einem der vorstehenden Ansprüche, **dadurch gekennzeichnet, daß** man mindestens 0,2 Stunden bei der erhöhten Temperatur nachbehandelt.

**8.** Verfahren nach einem der vorstehenden Ansprüche, **dadurch gekennzeichnet, daß** man pro Minute 0,05 - 2 % bezüglich des Volumens an o-Toluidin an Formaldehyd in die Reaktionsmischung einleitet.

**9.** Verfahren nach einem der vorstehenden Ansprüche, **dadurch gekennzeichnet, daß** man die Reaktionsmischung mit einer Geschwindigkeit von 1 - 6 m/sec im Kreislauf bewegt.

**10.** Verfahren nach einem der vorstehenden Ansprüche, **dadurch gekennzeichnet, daß** die Einspeisung des Formaldehyd in die Reaktionsmischung in einem Mischorgan, ausgewählt aus Reaktionsmischpumpe und Düse, erfolgt.

**11.** Verfahren nach Anspruch 10, **dadurch gekennzeichnet, daß** die bei der Einspeisung des Formaldehyd in die Reaktionsmischung in der Mischzone des Mischorgans lokal dissipierte Mischenergie 100 bis 100 000 W/l beträgt.
